(19) Europäisches Patentamt
European Patent Office
Office européen des brevets

(11) EP 2 838 495 B1

(12) EUROPEAN PATENT SPECIFICATION

(45) Date of publication and mention of the grant of the patent:
**23.01.2019 Bulletin 2019/04**

(21) Application number: **13730640.3**

(22) Date of filing: **17.04.2013**

(51) Int Cl.:
***A61K 8/33*** (2006.01) ***A61K 8/34*** (2006.01)
***A61K 8/35*** (2006.01) ***A61Q 19/04*** (2006.01)

(86) International application number:
**PCT/IB2013/001088**

(87) International publication number:
**WO 2013/156859 (24.10.2013 Gazette 2013/43)**

(54) **SELF-TANNING COSMETIC**

SELBSTBRÄUNUNGSKOSMETIKUM

PRODUIT COSMÉTIQUE DE TYPE AUTOBRONZANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.04.2012 US 201261636093 P**

(43) Date of publication of application:
**25.02.2015 Bulletin 2015/09**

(73) Proprietor: **Kao Corporation**
**Chuo-ku**
**Tokyo 103-8210 (JP)**

(72) Inventors:
- **INOUE, Michiaki**
  **Tokyo 131-8501 (JP)**
- **NOMIZU, Kayoko**
  **Tokyo 131-8501 (JP)**
- **YABUKI, Masayuki**
  **Tokyo 131-8501 (JP)**
- **KATO, Aya**
  **Tokyo 131-8501 (JP)**

(74) Representative: **Hoffmann Eitle**
**Patent- und Rechtsanwälte PartmbB**
**Arabellastraße 30**
**81925 München (DE)**

(56) References cited:
**WO-A1-02/096371**
**WO-A2-2013/171535**
**US-A- 6 086 903**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 838 495 B1

## Description

[Technical Field]

The present invention relates to a self-tanning cosmetic (a sunless tanning cosmetic).

[Background Art]

In North America and Europe, healthy sun tanned bronze skin is popular. However, the suntan caused by sun bathing or tanning bed raises concerns as possible causes of skin cancer and freckles due to the adverse effects caused by ultraviolet rays. For this reason, self-tanning cosmetics, which render a natural healthy suntan-like brown color effect to the skin, have been favorably used. Self-tanning cosmetics work to color the skin by the reaction of the skin and dihydroxyacetone (1,3-dihydroxy-2-propanone) contained in the cosmetic composition thereof. However, during this reaction process, an unpleasant odor, i.e., a reaction odor, is generated and causes discomfort to consumers upon application, which has been problematic.

However, substantially no study has been reported so far regarding the unpleasant odor upon application of a self-tanning cosmetic and the technology to improve the odor is in demand. Routinely, as measures to eliminate the discomfort caused by such unpleasant odor, the perfume concentration of a perfume composition may be increased or a perfume composition having an intense fragrance may be used. However, as a result, a stimulating odor derived from perfume substances results, posing a problem of deteriorating the quality of the fragrance.

It is known that such an unpleasant odor varies in a time dependent manner as the skin coloring caused by dihydroxyacetone progresses (Non-Patent Literature 1).

Also, Patent Literature 1 discloses a personal treatment composition containing a perfume composition containing 70% or more of an enduring perfume for a long lasting fragrance. Further, a self-tanning composition is disclosed as an example of such a personal treatment composition. Patent Literature 2 also discloses a self-tanning cosmetic scented with a perfume composition. The perfume composition applied in a self-tanning cosmetic contains several tens kinds of perfume raw materials. Meanwhile, Patent Literature 3 describes a perfume
capsule which can be used in a skin cosmetic and illustrates an example of a perfume which can neutralize the unpleasant odor when added to the perfume capsule. WO 02/096371 is concerned with the problem of neutralizing the malodor caused by compounds generated on the skin when dihydroxyacetone is applied on it and discloses the use of a rosemary extract for this purpose.

[Citation List]

[Non-Patent Literature]

[Non-Patent Literature 1] D. M. Hindenlang and M. E. McDonnell, Cosmetics & Toiletries magazine, 2008, Vol. 123, No. 7, p 67-74

[Patent Literature]

[Patent Literature 1] US6,086,903
[Patent Literature 2] US2011/0152383
[Patent Literature 3] EP1719554

[Summary of Invention]

The present invention provides a self-tanning cosmetic comprising the following component (A), component (B) and component (C):

(A) dihydroxyacetone;
(B) at least one compound selected from the group consisting of 4-methyl-3-decen-5-ol, 2-ethyl-4-(2,2,3-trimethyl-3-cyclopenten-1-yl)-2-buten-l-ol, 2-hexyl-3-phenyl-2-propenal and 2-methyl-3-(4-isopropylphenyl)propanal; and
(C) at least one compound selected from the group consisting of 3,7-dimethyl-1,6-nonadien-3-ol, 1-(2,2,6-trimethyl-2-cyclohexenyl)-2-buten-1-one, 1-(5,5-dimethyl-1-cyclohexen-1-yl)-4-penten-1-one, 1-(3,3-dimethyl-1-cyclohexen-1-yl)-4-penten-1-one, benzaldehyde and gamma-nonalactone.

The present invention further provides a self-tanning method reducing odor derived from a compound represented by the following formula (1), comprising a step of application of a self-tanning cosmetic to the skin for a browning reaction, wherein the self-tanning cosmetic comprises the component (A), the component (B) and the component (C):

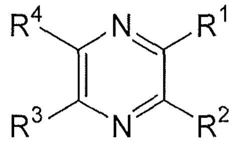

(1)

wherein $R^1$ represents a methyl group, an ethyl group or an acetyl group, and $R^2$, $R^3$ and $R^4$ each independently represent a hydrogen atom or a methyl group.

[Brief Description of Drawings]

Fig. 1 is a graph showing GC-MS analysis results of volatile substances generated by reacting human skin keratin and dihydroxyacetone.
Fig. 2 is a diagram showing a method for collecting the volatile substances generated from an arm to which a self-tanning agent was applied.
Fig. 3 is a graph showing the GC-MS analysis results of the volatile substances generated from the arm to which a self-tanning agent was applied.
Fig. 4 is a graph showing responses of olfactory receptor OR5K1 against various concentrations of 2,6-dimethylpyrazine (n = 5 and error bar = ±SE).
Fig. 5 is a graph showing responses of olfactory receptor OR5K1 against various concentrations of pyrazine compounds (n = 3 to 5).

[Description of Embodiments]

The present invention relates to a self-tanning cosmetic imparted with a good masking ability against the bad odor upon application of the self-tanning cosmetic without increasing the amount of a perfume added to the self-tanning cosmetic or using a perfume having an intense fragrance.

The description of Non-Patent Literature 1 described earlier did not specify the odor substance generated when staining the skin by dihydroxyacetone. Also, the self-tanning cosmetics of Patent Literature 1 and Patent Literature 2 provided an insufficient deodorizing effect against the earthy odor perceived as an unpleasant odor, which is generated particularly from the initial stage to middle stage of the reaction while a consumer is using the self-tanning cosmetic.

The present inventors found that pyrazines such as dimethylpyrazine are largely involved with the earthy odor generated while a consumer is using a self-tanning cosmetic. The present inventors further identified an olfactory receptor which senses the odor of pyrazines and found that specific several perfumes can be used as antagonists against the olfactory receptor. Furthermore, the present inventors found several perfumes which can effectively mask the odor of pyrazines which vaporizes from the self-tanning cosmetics. Based on the above findings, the present inventors found that a good masking ability against the bad odor generated upon application of a self-tanning cosmetic can be imparted by adding the above two groups of perfumes together with dihydroxyacetone to the self-tanning cosmetic, without increasing the amount of perfumes or using a perfume having an intense fragrance.

<Component (A)>

The self-tanning cosmetic of the present invention contains the component (A) dihydroxyacetone as an active ingredient to color by reacting with the skin. The content of component (A) in the self-tanning cosmetic of the present invention is, in the light of the coloring intensity to the skin, preferably 0.01 to 10 mass%, more preferably 0.01 to 7 mass%, even more preferably 0.01 to 5 mass%.

<Component (B)>

The component (B) of the present invention is a compound having a high antagonist activity against the olfactory receptor of pyrazines generated by the reaction of the component (A) dihydroxyacetone and the skin, and can reduce the bad odor derived from pyrazines. Used as the component (B) is at least one compound selected from the group

consisting of 4-methyl-3-decen-5-ol (Undecavertol ®; Givaudan), 2-ethyl-4-(2,2,3-trimethyl-3-cyclopenten-1-yl)-2-buten-1-ol (Bangalol ®; Givaudan), 2-hexyl-3-phenyl-2-propenal (also known as Amyl cinnamic aldehyde) and 2-methyl-3-(4-iso-propylphenyl)propanal (also known as Cyclamen aldehyde). The component (B) may be any one selected from the above listing, but preferably contains two or more compounds.

The content of component (B) in the self-tanning cosmetic of the present invention is, in light of the deodorizing effect and fragrance quality, preferably 0.001 to 0.1 mass%, more preferably 0.001 to 0.05 mass%, even more preferably 0.001 to 0.03 mass%, even more preferably 0.005 to 0.025 mass%, even more preferably 0.005 to 0.02 mass%, even more preferably 0.01 to 0.02 mass%.

<Component (C)>

The component (C) of the present invention is a favorable component having a good masking ability against the bad odor derived from pyrazines generated by the reaction of the component (A) dihydroxyacetone with the skin. Preferably used as the component (C) is at least one compound selected from the group consisting of 3,7-dimethyl-1,6-nonadien-3-ol (Ethyl linalool; Givaudan), 1-(2,2,6-trimethyl-2-cyclohexenyl)-2-buten-1-one (Damascone Alpha; Firmenich), 1-(5,5-dimethyl-1-cyclohexen-1-yl)-4-penten-1-one (Dynascone ®; Firmenich), 1-(3,3-dimethyl-1 -cyclohexen-1-yl)-4-penten-1-one (Dynascone ®; Firmenich), benzaldehyde and gamma-nonalactone (Aldehyde C-18 (so-called)). The component (C) may be any one selected from the above listing, but preferably contains two or more compounds.

The content of component (C) in the self-tanning cosmetic of the present invention is, in light of a deodorizing effect and fragrance quality, preferably 0.001 to 0.1 mass%, more preferably 0.003 to 0.1 mass%, even more preferably 0.005 to 0.1 mass%, even more preferably 0.01 to 0.1 mass%.

Further, the mass ratio of the component (B) content and the component (C) content in the self-tanning cosmetic of the present invention is, in light of enhancing the masking effect, preferably 1:10 to 10:1, more preferably 1:5 to 5:1, even more preferably 1:5 to 3:1.

<Component (D) and other perfume components>

Further, the self-tanning cosmetic of the present invention may contain, in addition to the components (B) and (C), known perfume raw materials in light of enhancing the product acceptability. The exemplary known perfume raw materials herein are the perfume raw materials described in Common Fragrance and Flavor Materials: Preparation, Properties and Uses, by Horst Surburg, Johannes Panten (John Wiley & Sons, 5th edition, 2006), Perfume and Flavor Chemicals, volume 1 and 2, by Steffen Arctander, (published by the author in 1969), and Perfume and Flavor Materials of Natural Origin, by Steffen Arctander (published by the author in 1961).

Unlimited examples of the known perfume raw material include 3,7-dimethyl-6-octen-1-ol (also known as Citronellol), 2,6-dimethyl-7-octen-2-ol (also known as Dihydro myrcenol), 3,7-dimethyl-1, 6-octadien-3-ol (also known as Linalool), phenyl ethyl alcohol, 3-(3,4-methylenedioxyphenyl)-2-methylpropanal (also known as Helional ®; IFF), p-tert-butyl-alpha-methyl hydrocinnamic aldehyde (also known as Lilial ®Givaudan), 1,3,4,6,7,8-hexahydro-4,6,6,7,8,8-hexamethyl cyclopenta-gamma-2-benzopyran (also known as Galaxolide ®; IFF), 4-(2,6,6-trimethyl-2-cyclohexen-1-yl)-3-methyl-3-buten-2-one (also known as Methyl ionone-gamma), 2-methylbutoxyacetic acid 2-propenyl ester (also known as Allyl amyl glycolate), decahydro-3a,6,6,9a-tetramethyl naphtha[2.1-b]furan (also known as Ambroxan ®; Kao), methyl dihydrojasmonate, benzyl acetate, gamma-undecalactone (Aldehyde C-14 (so-called)), cis-3-hexenyl salicylate, 7-methyl-3,4-dihydro-(2H)-1,5-benzodioxepin-3-one (Calone), and the like.

Further, these known perfume raw materials may be added to the cosmetic after being dissolved in a solvent. As the solvent for perfumes, dipropylene glycol (DPG), isopropyl myristate (IPM), triethyl citrate (TEC), or the like, may be used.

The total content of the component (B), the component (C) and other perfume components in the self-tanning cosmetic of the present invention, i.e., the content of all perfume components, is preferably 0.01 to 1.0 mass%, more preferably 0.1 to 0.7 mass%, even more preferably 0.2 to 0.7 mass%, even more preferably 0.3 to 0.6 mass%.

<Component (E) Polysaccharide>

Further, the self-tanning cosmetic of the present invention preferably contains a polysaccharide as the component (E) in light of reducing the reaction odor. Examples of such a polysaccharide include starch, cyclodextrin, powder cellulose, etc. The content of component (E) in the self-tanning cosmetic of the present invention is preferably 0.1 to 10 mass%, more preferably 1 to 10 mass%, even more preferably 2 to 8 mass%, even more preferably 3 to 7 mass%.

<Component (F) Erythrulose>

Further, the self-tanning cosmetic of the present invention preferably contains erythrulose as the component (F). In this instance, in light of coloring the skin, the component (A) dihydroxyacetone is preferably added in a higher concentration than the component (F) erythrulose. Further, the total content of the components (A) and (B) in the self-tanning cosmetic of the present invention is preferably 0.01 to 20 mass%, more preferably 0.01 to 15 mass%, even more preferably 0.01 to 10 mass%, even more preferably 0.01 to 7 mass%, even more preferably 0.01 to 5 mass%.

<Other components>

To the self-tanning cosmetic of the present invention, various routinely used components may be added. Examples include a moisturizer, an emulsifier, an antiinflammatory agent, a preservative, an oil, a thickener, a pH adjuster, water, or the like.

<Dosage form>

The dosage form for the self-tanning cosmetic of the present invention may be any form insofar as it is acceptable as a cosmetic. Examples include a spray, a mist, a cream, a lotion, a gel, a powder, a mask, a solution, an emulsion, or the like. A cream form is particularly desirable.

<Method for reducing pyrazine-derived bad odor generated by the browning reaction>

The present invention can reduce the pyrazines-derived odor, which is generated by the application of the cosmetic containing the component (A) to the skin and the proceeding of browning reaction, by adding the component (B) and the component (C) to the cosmetic containing the component (A). The pyrazines are the compounds represented by the following formula (1):

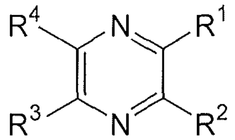

(1)

wherein $R^1$ represents a methyl group, an ethyl group or an acetyl group, and $R^2$, $R^3$ and $R^4$ each independently represent a hydrogen atom or a methyl group,
which are in the compound group extremely analogous in the chemical structure to the compounds which the present inventors revealed are the causative substances of the tanning odor. Examples of the causative substances of the tanning odor revealed by the present inventors include 2-methyl pyrazine, 2,6-dimethylpyrazine, 2,5-dimethylpyrazine, 2-ethyl-6-methyl pyrazine, 2-acetyl-3-methyl pyrazine, 2,3,6-trimethyl pyrazine, 2-acetyl-3,5-dimethylpyrazine, and the like; particularly with 2,6-dimethylpyrazine and 2,5-dimethylpyrazine being frequently detected. These pyrazine compounds are the well known compounds as the flavors for food products. However, they are not well known as the structural components of the tanning odor.

The kind and amount to be used of each component for the method of the present invention is the same as the contents described earlier for the self-tanning cosmetics.

The self-tanning cosmetic of the present invention is preferably applied to the skin in an amount of 0.005 to 0.01 g/$cm^2$. The part of skin to which the cosmetic is applied is not particularly limited, and the cosmetic is applicable, for example, to the arms, legs, back, nape of the neck, face, and the like.

Regarding the above-mentioned embodiments, hereafter preferable embodiments of the present invention are further disclosed.

<1> A self-tanning cosmetic comprising the following component (A), component (B) and component (C):

(A) dihydroxyacetone;
(B) at least one or more compounds selected from the group consisting of 4-methyl-3-decen-5-ol, 2-ethyl-4-(2,2,3-trimethyl-3-cyclopenten-1-yl)-2-buten-1-ol, 2-hexyl-3-phenyl-2-propenal and 2-methyl-3-(4-isopropylphenyl)propanal; and
(C) at least one or more compounds selected from the group consisting of 3,7-dimethyl-1,6-nonadien-3-ol, 1-(2,2,6-trimethyl-2-cyclohexenyl)-2-buten-1-one, 1-(5,5-dimethyl-1-cyclohexen-1-yl)-4-penten-1-one, 1-(3,3-dimethyl-1-cyclohexen-1-yl)-4-penten-1-one, benzaldehyde and gamma-nonalactone.

<2> The self-tanning cosmetic of the above-mentioned item <1>, wherein the mass ratio of the contents of the component (B) and the component (C) is preferably from 10:1 to 1:10, more preferably from 1:5 to 5:1, even more preferably from 1:5 to 3:1.
<3> The self-tanning cosmetic of the above-mentioned item <1> or <2>, wherein the content of the component (B) is preferably from 0.001 to 0.1 mass%, more preferably from 0.001 to 0.05 mass%, even more preferably from 0.001 to 0.03 mass%, even more preferably from 0.005 to 0.025 mass%, even more preferably from 0.005 to 0.02 mass%, even more preferably from 0.01 to 0.02 mass%.
<4> The self-tanning cosmetic of any one of the above-mentioned items <1> to <3>, wherein the content of the component (C) is preferably from 0.001 to 0.1 mass%, more preferably from 0.003 to 0.1 mass%, even more preferably from 0.005 to 0.1 mass%, even more preferably from 0.01 to 0.1 mass%.
<5> The self-tanning cosmetic of any one of above-mentioned items <1> to <4>, wherein the content of the component (A) is preferably from 0.01 to 10 mass%, more preferably from 0.01 to 7 mass%, even more preferably from 0.01 to 5 mass%.
<6> The self-tanning cosmetic of any one of the above-mentioed items <1> to <5>, further comprising a polysaccharide as a component (E).
<7> The self-tanning cosmetic of the above-mentioned item <6>, wherein the content of the component (E) is preferably from 0.1 to 10 mass%, more preferably from 1 to 10 mass%, even more preferably from 2 to 8 mass%, even more preferably from 3 to 7 mass%.
<8> The self-tanning cosmetic of any one of the above-mentioned items <1> to <7>, further comprising erythrulose as a component (F).
<9> The self-tanning cosmetic of the above-mentioned item <8>, wherein the total content of the components (A) and (F) is preferably from 0.01 to 20 mass%, more preferably from 0.01 to 15 mass%, even more preferably from 0.01 to 10 mass%, even more preferably from 0.01 to 7 mass%, even more preferably from 0.01 to 5 mass%.
<10> A self-tanning method reducing odor derived from a compound represented by the following formula (1) and generated by the application of a cosmetic containing the following component (A) to skin and the proceeding of tanning reaction, comprising adding the following component (B) and component (C) to the cosmetic:

(A) dihydroxyacetone;
(B) at least one or more compounds selected from the group consisting of 4-methyl-3-decen-5-ol, 2-ethyl-4-(2,2,3-trimethyl-3-cyclopenten-1-yl)-2-buten-1-ol, 2-hexyl-3-phenyl-2-propenal and 2-methyl-3-(4-isopropylphenyl)propanal; and
(C) at least one or more compounds selected from the group consisting of 3,7-dimethyl-1,6-nonadien-3-ol, 1-(2,2,6-trimethyl-2-cyclohexenyl)-2-buten-1-one, 1-(5,5-dimethyl-1-cyclohexen-1-yl)-4-penten-1-one, 1-(3,3-dimethyl-1-cyclohexen-1-yl)-4-penten-1-one, benzaldehyde and gamma-nonalactone;

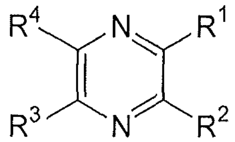

(1)

wherein $R^1$ represents a methyl group, an ethyl group or an acetyl group, and $R^2$, $R^3$ and $R^4$ each independently represent a hydrogen atom or a methyl group.

<11> The method of the above-mentioned item <10>, wherein the odor derived from the compound represented by the formula (1) is preferably earthy odor.
<12> The method of the above-mentioned item <10> or <11>, the mass ratio of the contents of the component (B) and the component (C) in the cosmetic is preferably from 10:1 to 1:10, more preferably from 1:5 to 5:1, even more preferably from 1:5 to 3:1.
<13> The method of any one of the above-mentioned items <10> to <12>, wherein the content of the component (B) in the cosmetic is preferably from 0.001 to 0.1 mass%, more preferably from 0.001 to 0.05 mass%, even more preferably from 0.001 to 0.03 mass%, even more preferably from 0.005 to 0.025 mass%, even more preferably from 0.005 to 0.02 mass%, even more preferably from 0.01 to 0.02 mass%.
<14> The method of any one of the above-mentioned items <10> to <13>, wherein the content of the component (C) in the cosmetic is preferably from 0.001 to 0.1 mass%, more preferably from 0.003 to 0.1 mass%, even more preferably from 0.005 to 0.1 mass%, even more preferably from 0.01 to 0.1 mass%.

<15> The method of any one of the above-mentioned items <10> to <14>, wherein the content of the component (A) in the cosmetic is preferably from 0.01 to 10 mass%, more preferably from 0.01 to 7 mass%, even more preferably from 0.01 to 5 mass%.

<16> Use of a composition comprising the following component (A), component (B) and component (C) as a self-tanning cosmetic:

(A) dihydroxyacetone;
(B) at least one or more compounds selected from the group consisting of 4-methyl-3-decen-5-ol, 2-ethyl-4-(2,2,3-trimethyl-3-cyclopenten-1-yl)-2-buten-1-ol, 2-hexyl-3-phenyl-2-propenal and 2-methyl-3-(4-isopropylphenyl)propanal; and
(C) at least one or more compounds selected from the group consisting of 3,7-dimethyl-1,6-nonadien-3-ol, 1-(2,2,6-trimethyl-2-cyclohexenyl)-2-buten-1-one, 1-(5,5-dimethyl-1-cyclohexen-1-yl)-4-penten-1-one, 1-(3,3-dimethyl-1-cyclohexen-1-yl)-4-penten-1-one, benzaldehyde and gamma-nonalactone.

<17> The use of the above-mentioned item <16>, wherein the mass ratio of the contents of the component (B) and the component (C) in the composition is preferably from 10:1 to 1:10, more preferably from 1:5 to 5:1, even more preferably from 1:5 to 3:1.

<18> The use according to the above-mentioned item <16> or <17>, wherein the content of the component (B) in the composition is preferably from 0.001 to 0.1 mass%, more preferably from 0.001 to 0.05 mass%, even more preferably from 0.001 to 0.03 mass%, even more preferably from 0.005 to 0.025 mass%, even more preferably from 0.005 to 0.02 mass%, even more preferably from 0.01 to 0.02 mass%.

<19> The use according to any one of the above-mentioned items <16> to <18>, wherein the content of the component (C) in the composition is preferably from 0.001 to 0.1 mass%, more preferably from 0.003 to 0.1 mass%, even more preferably from 0.005 to 0.1 mass%, even more preferably from 0.01 to 0.1 mass%.

<20> The use according any one of the above-mentioned items <16> to <19>, wherein the content of the component (A) in the composition is preferably from 0.01 to 10 mass%, more preferably from 0.01 to 7 mass%, even more preferably from 0.01 to 5 mass%.

<21> The use according to any one of the above-mentioned items <16> to <20>, wherein the composition preferably further comprises a polysaccharide as a component (E).

<22> The use according to any one of the above-mentioned items <21>, wherein the content of the component (E) in the composition is preferably from 0.1 to 10 mass%, more preferably from 1 to 10 mass%, even more preferably from 2 to 8 mass%, even more preferably from 3 to 7 mass%.

<23> The use according to any one of the above-mentioned items <16> to <22>, wherein the composition preferably further comprises erythrulose as a component (F).

<24> The use according to the above-mentioned items <23>, wherein the total content of the components (A) and (F) in the composition is preferably from 0.01 to 20 mass%, more preferably from 0.01 to 15 mass%, even more preferably from 0.01 to 10 mass%, even more preferably from 0.01 to 7 mass%, even more preferably from 0.01 to 5 mass%.

[Example]

The following examples further describe and demonstrate embodiments of the present invention. The examples are given solely for the purpose of illustration and are not to be construed as limitations of the present invention.

Test Example 1: Analysis of the volatile substance generated by the reaction of dihydroxyacetone and the skin keratin

The analysis of the volatile substances generated by the reaction of dihydroxyacetone and the skin keratin was carried out.

A subject (one healthy Japanese male) refrained from applying any kind of cream to his heels on the day before the keratin-collection. After the subject thoroughly washed the heels with warm water and completely dried, the calloused skin (powdery form) of the heels was collected using a commercial keratin smoother (Motorized heel smoother, Panasonic ES2502OPP). After the collection, the collected powder was put in a glass container and, without a lid, left to stand overnight in a dryer (50°C), thereby obtaining a dehydrated substantially odorless white powder. The thus dried keratin powder, in a glass tube with the lid on, was stored in a refrigerator unless it was used.

10 mg of the dried keratin powder and 0.1 mL of an aqueous solution of dihydroxyacetone (10 mass%) were mixed in a glass container (20 mL). The upper part of the container was sealed with a rubber septum. The mixture was stored for 5 days while heated (50°C). As a result, the color of keratin powder changed to brown and an intense reaction odor was generated. The rubber septum was pierced with an SPME (Solid Phase Micro Extraction) fiber, and the volatile substances generated were collected and analyzed for GC-MS. The results are shown in Fig. 1.

As shown in Fig. 1, lower aldehydes, lower fatty acids, furans, pyrans, pyrazines, and the like, were detected. In the low threshold components, 2,6-dimethylpyrazine had a particularly high peak abundance.

The same test (stored for 5 days while heated to 50°C) was carried out using 0.1 mL of an aqueous solution of dihydroxyacetone (10 mass%) or a mixture of 10 mg of keratin powder and 0.1 mL of pure water, but no pyrazines were detected.

Test Example 2: Analysis of volatile substances generated during the self-tanning agent application and evaluation of the contribution of the substance to odor

Five healthy American females (all Caucasian/white) cooperated with the test. The subjects (with their right hand) applied 0.5 mL of the self-tanning agent (unperfumed, dihydroxyacetone concentration 2 mass%, erythrulose concentration 0.5 mass%) throughout their entire left forearm.

The collection of reaction odor was carried out by a technique to which the SPME (Solid Phase Micro Extraction) method was applied. Fig. 2 shows a diagram of the technique.

First, a large number of air holes (inner diameter 4 mm) were made at the tip (4 cm) of a commercial Teflon tube (inner diameter 8 mm, outer diameter 10 mm, length 9 cm). Using 2 circular inactive rubber septa, the SPME needle part was fixed inside the Teflon tube. Subsequently, the SPME fiber (originally stored inside the needle) coated with an adsorbent was pushed out and exposed. The connecting part (notch) to the SPME holder (main body) was covered and protected with a thin Teflon tube (6 cm).

For a sleeve (arm cover), one with the surface coated with Teflon (FLON INDUSTRY, 120 mmϕ, length 400 mm) was used. After the coated surface was turned inside (facing the arm), the SPME device was arranged in the center and fixed from outside with 2 plastic rings. The SPME fiber used was P/N 57335-U (manufactured by SUPELCO Inc.) up to 4 hours from immediately after the application of the self-tanning agent and P/N 57348-U (manufactured by SUPELCO Inc.) from 4 hours to 8 hours after the application of the self-tanning agent. When the fiber was exchanged, the Teflon sleeve was also exchanged with a new one.

(Analysis of the volatile substances generated from the arm)

Fig. 3 shows an example of the GC-MS analysis results (analysis results of the substances collected from the arm from 4 to 8 hours after the application). Most of the high peaks were made by the silicon compounds derived from the SPME fiber. However, detailed analysis close to the baseline revealed the presence of lower ketone, lower alcohol, 2,6-dimethylpyrazine, acetic acid, formic acid, and the like. The analysis results of the volatile substances collected from the arm of the same subject from 0 to 4 hours after the application were similar to the results analyzed from 4 to 8 hours after the application. In the head space analysis of the arms to which the agent was not applied, 2,6-dimethylpyrazine was not detected.

(Evaluation of contribution of the substance to the odor)

The odor of 5 subjects' arm was subjected to sensory evaluation. The table below shows the evaluation results on the similarity of the substances easily detected directly from the arm after the product application to the reaction odor. The evaluation was carried out using reagents by 4 people.

[Criteria]

A: Evaluated as similar by 3 or more people
B: Evaluated as similar by 1 to 2 people
C: Evaluated as similar by 0 people

[Table 1]

| Substance | Odor similarity |
|---|---|
| Acetaldehyde | C |
| 2-Propanone | C |
| 2-Butanone | C |
| 2-Propanol | C |

(continued)

| Substance | Odor similarity |
|---|---|
| 1-Butanol | C |
| 2,6-Dimethyl pyrazine | A |
| 2-Methyl pyrazine | A |
| 2,5-Dimethyl pyrazine | A |
| Acetic acid | B |
| Formic acid | C |

Test Example 3 Identification of olfactory receptor responding to pyrazine derivatives

1) Cloning of human olfactory receptor gene

Human olfactory receptors were cloned based on sequence information registered in GenBank by PCR using human genomic DNA female (G1521: Promega Corporation) as a template. Each gene amplified by PCR was incorporated into pENTR vector (Invitrogen Inc.) in accordance with the manual and recombined to the NotI and AscI sites located downstream of a Flag-Rho tag sequence in a pME18S vector using the NotI and AscI sites present on the pENTR vector.

2) Production of pME18S-RTP1S vector

An RTP1S variant gene (SEQ ID NO: 3) encoding an RTP1S variant (SEQ ID NO: 4) was incorporated into the EcoRI and XhoI sites of a pME18S vector.

3) Production of olfactory receptor-expressing cell

HEK293 cells expressing 373 types of human olfactory receptors were produced. A reaction solution having a composition shown in Table 2 was prepared, left to stand in a clean bench for 15 min and then was dispensed in each well of a 96 well plate (Becton, Dickinson and Company) . Subsequently, HEK293 cells (100 μL, 3 x $10^5$ cells/$cm^2$) were seeded in each well and cultured in an incubator at 37°C under 5 mass% $CO_2$ for 24 hours.

[Table 2]

| | |
|---|---|
| OPTI-MEM (GIBCO) | 50 μL |
| Human olfactory receptor gene (incorporated in a pME18S vector having a Flag-Rho tag at the N terminal) | 0.075 μg |
| pGL4.29 (fluc2P-CRE-hygro, Promega) | 0.03 μg |
| pGL4.75 (hRluc-CMV, Promega) | 0.03 μg |
| pME18S-RTP1S variant vector | 0.03 μg |
| lipofectamine 2000 (Invitrogen) | 0.4 μL |

4) Luciferase assay

An olfactory receptor expressed in HEK293 cells couples with endogenous Gas in the cells to activate adenylate cyclase and thereby increases the amount of intracellular cAMP. In this study, the odor response was measured by luciferase reporter gene assay which monitors an increase in amount of intracellular cAMP as the luminescence value derived from a firefly luciferase gene (fluc2P-CRE-hygro). A Renilla luciferase gene was fused with the downstream of a CMV promoter (hRluc-CMV) and was also introduced to HEK293 cells as an internal standard to correct errors in gene transfer efficiency and number of cells.

The culture medium was removed from the culture produced in the above 3), and 75 μL of a solution prepared with a CD293 medium (Invitrogen Inc.) so as to contain a pyrazine derivative (1 mM 2,6-dimethylpyrazine) was added thereto. The cells were cultured in a $CO_2$ incubator for 2.5 hours to sufficiently express the luciferase gene in the cells.

The luciferase activity was measured with a Dual-Glo™ luciferase assay system (Promega Corporation) in accordance with the operating manual of the system. The fold increases were calculated by dividing the luminescence value derived from firefly luciferase induced by stimulation with the odor substances by the luminescence value in cells not stimulated with the odor substances and were used as an index of response strength.

5) Result

The response of each of the 373 types of olfactory receptors to 2,6-dimethylpyrazine (1 mM) was measured, and the results showed that only olfactory receptor OR5K1 responded to 2,6-dimethylpyrazine (Fig. 4).
Olfactory receptor OR5K1 is expressed in human olfactory cells and has been registered in GenBank under the accession number GI: 115270955. OR5K1 is a protein encoded by a gene having a nucleotide sequence represented by SEQ ID NO: 1 and having the amino acid sequence represented by SEQ ID NO: 2.
Response of OR5K1 to 2,6-dimethylpyrazine has not been reported until now, and OR5K1 is a novel 2,6-dimethylpyrazine receptor.

Test Example 4 Response characteristics of OR5K1 to pyrazine derivatives

Olfactory receptor OR5K1 (SEQ ID NO: 2) was expressed in HEK293 cells together with an RTP1S variant (SEQ ID NO: 4) by the same procedure as in Test Example 3, and the dependency of the response on the concentration (0, 3, 10, 30, 100, 300, and 1000 μM) in various pyrazine compounds was investigated. The pyrazine compounds used in this Example were pyrazine and pyrazine derivatives selected from the group consisting of 2-methylpyrazine, 2,3-dimethylpyrazine, 2,5-dimethylpyrazine, 2,3,5-trimethylpyrazine, 2,3,5,6-tetramethylpyrazine, and 2-ethylpyrazine.
The results were that OR5K1 concentration-dependently responded to the pyrazine derivatives, i.e., 2-methylpyrazine, 2,3-dimethylpyrazine, 2,5-dimethylpyrazine, 2,6-dimethylpyrazine, 2,3,5-trimethylpyrazine, 2,3,5,6-tetramethylpyrazine, and 2-ethylpyrazine, but did not respond to pyrazine (Fig. 5).

Test Example 5 Identification of antagonist against OR5K1

Antagonist activities of 174 test materials against olfactory receptor OR5K1 were investigated by examining response of OR5K1 to a pyrazine derivative.
2,6-Dimethylpyrazine (1 mM) and each of the test materials (300 μM) were added to HEK293 cells expressing olfactory receptor OR5K1 by the same procedure as in Test Example 4, and the response of the olfactory receptor to 2,6-dimethylpyrazine was measured to evaluate a change in response of the receptor due to addition of the test material. Perfumes recognized to have cytotoxicity were re-evaluated using a mixture of 333 μM of 2,6-dimethylpyrazine and 100 μM of a test material.
The receptor response-inhibiting rate of a test material was calculated as follows. The receptor activity (X-Y) by stimulation with 2,6-dimethylpyrazine alone was determined by subtracting the luminescence value (Y) in cells to which the receptor was introduced but were not stimulated with 2,6-dimethylpyrazine from the luminescence value (X) derived from firefly luciferase induced by odor stimulation with 2,6-dimethylpyrazine alone. Similarly, the receptor activity (Z-Y) in the presence of a test material was determined by subtracting the luminescence value (Y) in cells not stimulated with 2,6-dimethylpyrazine from the luminescence value (Z) stimulated with a mixture of 2,6-dimethylpyrazine and the test material. The reduction rate of the receptor activity (Z-Y) in the presence of a test material to the receptor activity (X-Y) by stimulation with 2,6-dimethylpyrazine alone was calculated by the following computation expression:

$$\text{Inhibition rate } (\%) = \{1-(Z-Y)/(X-Y)\} \times 100,$$

to determine the receptor response-inhibiting rate of the test material. In the measurement, multiple independent experiments were performed in duplicate, and the average of each experiment was used.

<Result>

As shown in Table 3 regarding representative compounds, 4-methyl-3-decen-5-ol (Undecavertol ®; Givaudan), 2-ethyl-4- (2,2,3-trimethyl-3-cyclopenten-1-yl)-2-buten-1-ol (Bangalol ®; Givaudan), 2-hexyl-3-phenyl-2-propenal (also known as Amyl cinnamic aldehyde) and 2-methyl-3-(4-iso-propylphenyl)propanal (also known as Cyclamen aldehyde) recognized to have the antagonist activity on the response of OR5K1 to pyrazine derivatives.
On the other hand, methyl dihydrojasmonate, 4-methyl-2-(2-methylpropyl)tetrahydro-2H-4-pyranol (also known

as Florosa) and 4-(p-hydroxyphenyl)-2-butanone (also known as Raspberry ketone) recognized to have no antagonist activity described above.

[Table 3]

| Test material | Receptor response-inhibiting rate |
|---|---|
| Undecavertol | 75% or more |
| Bangalol | 75% or more |
| Amyl cinnamic aldehyde | 75% or more |
| Cyclamen aldehyde | 75% or more |
| Methyl dihydrojasmonate | Not inhibited |
| Florosa | Not inhibited |
| Raspberry ketone | Not inhibited |

Test Example 6 Sensory Test

<Preparation of compositions>

The unperfumed self-tanning cosmetic shown in Table 4 was prepared and 1 mass% of dimethylpyrazine was added thereto. Each of the perfume raw materials was added to the cosmetic so that a perfume concentration is 0.1 mass%, and the evaluation was carried out in accordance with the following evaluation method and criteria.

<Evaluation method and criteria>

(1) Evaluation method

1. Scent 99.9 g of the self-tanning cosmetic containing dimethylpyrazine with 0.1 g (0.1 mass%) of each of the perfume raw materials. At this time, in consideration of a threshold, specific raw materials were used in the form of a perfume solution diluted to 10 mass% with dipropylene glycol.
2. Uniformly spread 0.5 g of the prepared self-tanning cosmetic on a piece of wrapping paper sized 15 cm x 15 cm.
3. Evaluate the fragrance in accordance with the following criteria.

(2) Criteria

5: No malodor of dimethylpyrazine
4: Slight malodor of dimethylpyrazine
3: Moderate malodor of dimethylpyrazine
2: Slightly strong malodor of dimethylpyrazine
1: Strong malodor of dimethylpyrazine

<Evaluation result>

Table 5 shows the evaluation results of methyl dihydrojasmonate and 5 perfume raw materials which had good reduction activity on the discomfort caused by the bad odor as the result of the above evaluation. The evaluation was carried out based on the discussion among four specialized panelists and was shown in units of 0.5.

[Table 4]

| Ingredient | mass% |
|---|---|
| Water | 74.792 |

(continued)

| Ingredient | mass% |
|---|---|
| Waxy Corn Starch * | 3.750 |
| Glycerin | 7.000 |
| Methylparaben | 0.200 |
| Acrylates / C10-30 Alkyl Acrylate Crosspolymer | 0.300 |
| Cetearyl Alcohol | 3.300 |
| Stearic Acid | 0.500 |
| Petrolatum | 1.750 |
| Mineral Oil | 1.750 |
| Ceteareth-20 ** | 1.400 |
| Steareth-2 *** | 0.900 |
| Octyldodecyl Myristate | 0.500 |
| Propylparaben | 0.100 |
| Dibutylhydroxytoluene | 0.100 |
| Dimethicone (Gum) | 0.700 |
| Citric Acid | 0.018 |
| Erythrulose (80 mass%) | 0.500 |
| Dihydroxyacetone | 2.000 |
| Caramel | 0.040 |
| DMDM Hydantoin **** | 0.400 |
| Total | 100.000 |

* Proposed INCI Name. Purchased from Akzo Nobel as a Trade Name of PC-10-40.
** Ceteareth-20 is the polyethylene glycol ether of Cetearyl Alcohol, wherein the average chain length of polyethylene glycol unit is 20.
*** Steareth-2 is the polyethylene glycol ether of Stearyl Alcohol, wherein the average chain length of polyethylene glycol unit is 2.
**** DMDM Hydantoin: 1,3-Bis(hydroxymethyl)-5,5-dimethylhydantoin. This material exists as an equilibrium mixture with 1-Hydroxymethyl-5,5-dimethylhydantoin

[Table 5]

| Perfume raw material | Evaluation result |
|---|---|
| Ethyl linalool | 4.0 |
| α-Damascone | 4.0 |
| Dynascone * | 5.0 |
| Benzaldehyde | 4.5 |
| Aldehyde C-18 | 4.5 |
| Methyl dihydrojasmonate | 2.0 |

* Dynascone (purchased from Firmenich) : 10 mass% dipropylene glycol solution of the mixture of 1-(5,5-Dimethyl-1-cyclohexen-1-yl)-4-penten-1-one and 1-(3,3-Dimethyl-1-cyclohexen-1-yl)-4-penten-1-one

Examples 1 to 15, Comparative Examples 1 to 7

The self-tanning cosmetic shown in Table 4 was scented with 0.5 mass% of Perfumes 1 to 17 shown in Tables 6 and 7, and evaluated in accordance with the following method and criteria.

<Evaluation method and criteria>

(1) Evaluation method

0.5 g of the perfume-applied self-tanning cosmetic was weighed, mixed homogeneously with 0.1 g of keratin powder and uniformly spread on a piece of wrapping paper sized 15 cm x 15 cm. The resultant paper was left to stand at 25°C in a thermostat and the trailing fragrance from the wrapping paper was evaluated 3 hours later in accordance with the following criteria. The results are shown in Tables 8 and 9. The evaluation was carried out based on the discussion among four specialized panelists and was shown in units of 0.5.

(2) Criteria

5: No Malodor
4: Slight Malodor
3: Moderate Malodor
2: Slightly Strong Malodor
1: Strong Malodor

[Table 6]

| | | Perfume 1 | Perfume 2 | Perfume 3 | Perfume 4 | Perfume 5 | Perfume 6 | Perfume 7 | Perfume 8 | Perfume 9 | Perfume 10 | Perfume 11 |
|---|---|---|---|---|---|---|---|---|---|---|---|---|
| Component (B) | Undecavertol | 30 | 3 | 0.5 | 30 | 50 | 3 | | | 30 | 30 | 30 |
| | Bangalol | 6 | 2 | 2 | 2 | 2 | 30 | 2 | 6 | 2 | 2 | 2 |
| | Amyl Cinnamic Aldehyde | | 2 | 2 | 2 | 2 | 2 | 30 | | 2 | 2 | 2 |
| | Cyclamen Aldehyde | | 2 | 2 | 2 | 2 | 2 | | 40 | 2 | 2 | 2 |
| Component (C) | Ethyl Linalool | 30 | 30 | 30 | 30 | 30 | 30 | 30 | 30 | 25 | 50 | 100 |
| | a-Damascone | | | | | | | | | 2 | 2 | 2 |
| | Dynascone | | | | | | | | | 1 | 1 | 1 |
| | Benzaldehyde | | | | | | | | | 1 | 1 | 1 |
| | Aldehyde C-18 | | | | | | | | | 1 | 1 | 1 |
| Component (D) | Benzyl Acetate | 5 | 5 | 5 | 5 | 5 | 5 | 5 | 5 | 5 | 5 | 5 |
| | Dihydro Myrcenol | 10 | 10 | 10 | 10 | 10 | 10 | 10 | 10 | 10 | 10 | 10 |
| | Allyl Amyl Glycolate | 1 | 1 | 1 | 1 | 1 | 1 | 1 | 1 | 1 | 1 | 1 |
| | Calone | 12 | 1.2 | 1.2 | 1.2 | 1.2 | 1.2 | 1.2 | 1.2 | 1.2 | 1.2 | 1.2 |
| | Aldehyde C-14 peach | 1.5 | 1.5 | 1.5 | 1.5 | 1.5 | 1.5 | 1.5 | 1.5 | 1.5 | 1.5 | 1.5 |
| | Helional | 50 | 50 | 50 | 50 | 50 | 50 | 50 | 50 | 50 | 50 | 50 |
| | Phenyl Ethyl Alcohol | 10 | 10 | 10 | 10 | 10 | 10 | 10 | 10 | 10 | 10 | 10 |
| | Citronellol | 30 | 30 | 30 | 30 | 30 | 30 | 30 | 30 | 30 | 30 | 30 |
| | Lilial | 130 | 130 | 130 | 130 | 130 | 130 | 130 | 130 | 130 | 130 | 130 |
| | Linalool | 40 | 40 | 40 | 40 | 40 | 40 | 40 | 40 | 40 | 40 | 40 |
| | Methyl Dihydrojasmonate | 250 | 250 | 250 | 250 | 250 | 250 | 250 | 250 | 250 | 250 | 250 |
| | Ambroxan | 1.3 | 1.3 | 1.3 | 1.3 | 1.3 | 1.3 | 1.3 | 1.3 | 1.3 | 1.3 | 1.3 |
| | Galaxolide 651PM | 200 | 200 | 200 | 200 | 200 | 200 | 200 | 200 | 200 | 200 | 200 |
| | Methyl ionone G | 50 | 50 | 50 | 50 | 50 | 50 | 50 | 50 | 50 | 50 | 50 |
| | cis-3-Hexenyl Saticilate | 20 | 20 | 20 | 20 | 20 | 20 | 20 | 20 | 20 | 20 | 20 |
| | Dipropylene Glycol | 134 | 161 | 163.5 | 134 | 114 | 133 | 138 | 124 | 134 | 109 | 59 |
| Total | | 1000 | 1000 | 1000 | 1000 | 1000 | 1000 | 1000 | 1000 | 1000 | 1000 | 1000 |

[Table 7]

| | | Perfume 12 | Perfume 13 | Perfume 14 | Perfume 15 | Perfume 16 | Perfume 17 | Perfume 18 | Perfume 19 | Perfume 20 | Perfume 21 | Perfume 22 |
|---|---|---|---|---|---|---|---|---|---|---|---|---|
| Component (B) | Undecavertol | 30 | 30 | 30 | 30 | 30 | 30 | 60 | | | | |
| | Bangalol | 2 | 2 | 2 | 2 | 2 | 6 | 6 | | | | |
| | Amyl Cinnamic Aldehyde | 2 | 2 | 2 | 2 | 2 | | | | | | |
| | Cyclamen Aldehyde | 2 | 2 | 2 | 2 | 2 | | | | | | |
| Component (C) | Ethyl Linalool | 2 | 10 | 5 | 10 | | | | 30 | 70 | 25 | |
| | a-Damascone | 2 | 2 | 10 | | | | | | | 2 | |
| | Dynascone | 1 | 1 | 5 | 10 | | | | | | 1 | |
| | Benzaldehyde | 1 | 1 | 10 | 5 | | | | | | 1 | |
| | Aldehyde C-18 | 1 | 1 | | 5 | | | | | | 1 | |
| Component (D) | Benzyl Acetate | 5 | 5 | 5 | 5 | 5 | 5 | 5 | 5 | 5 | 5 | 5 |
| | Dihydro Myrcenol | 10 | 10 | 10 | 10 | 10 | 10 | 10 | 10 | 10 | 10 | 10 |
| | Allyl Amyl Glycolate | 1 | 1 | 1 | 1 | 1 | 1 | 1 | 1 | 1 | 1 | 1 |
| | Calone | 1.2 | 1.2 | 1.2 | 1.2 | 1.2 | 1.2 | 1.2 | 1.2 | 1.2 | 1.2 | 1.2 |
| | Aldehyde C-14 peach | 1.5 | 1.5 | 1.5 | 1.5 | 1.5 | 1.5 | 1.5 | 1.5 | 1.5 | 1.5 | 1.5 |
| | Helional | 50 | 50 | 50 | 50 | 50 | 50 | 50 | 50 | 50 | 50 | 50 |
| | Phenyl Ethyl Alcohol | 10 | 10 | 10 | 10 | 10 | 10 | 10 | 10 | 10 | 10 | 10 |
| | Citronellol | 30 | 30 | 30 | 30 | 30 | 30 | 30 | 30 | 30 | 30 | 30 |
| | Lilial | 130 | 130 | 130 | 130 | 130 | 130 | 130 | 130 | 130 | 130 | 130 |
| | Linalool | 40 | 40 | 40 | 40 | 40 | 40 | 40 | 40 | 40 | 40 | 40 |
| | Methyl Dihydrojasmonate | 250 | 250 | 250 | 250 | 250 | 250 | 250 | 250 | 250 | 250 | 250 |
| | Ambroxan | 1.3 | 1.3 | 1.3 | 1.3 | 1.3 | 1.3 | 1.3 | 1.3 | 1.3 | 1.3 | 1.3 |
| | Galaxolide 65IPM | 200 | 200 | 200 | 200 | 200 | 200 | 200 | 200 | 200 | 200 | 200 |
| | Methyl Ionone G | 50 | 50 | 50 | 50 | 50 | 50 | 50 | 50 | 50 | 50 | 50 |
| | cis-3-Hexenyl Salicilate | 20 | 20 | 20 | 20 | 20 | 20 | 20 | 20 | 20 | 20 | 20 |
| | Dipropylene Glycol | 157 | 149 | 134 | 134 | 164 | 164 | 134 | 170 | 130 | 170 | 200 |
| Total | | 1000 | 1000 | 1000 | 1000 | 1000 | 1000 | 1000 | 1000 | 1000 | 1000 | 1000 |

[Table 8]

| | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|
| Composition in Table (Basic formulation) | 99.5 | 99.5 | 99.5 | 99.5 | 99.5 | 99.5 | 99.5 | 99.5 | 99.5 | 99.5 | 99.5 |
| Perfume 1 | 0.5 | | | | | | | | | | |
| Perfume 2 | | 0.5 | | | | | | | | | |
| Perfume 3 | | | 0.5 | | | | | | | | |
| Perfume 4 | | | | 0.5 | | | | | | | |
| Perfume 5 | | | | | 0.5 | | | | | | |
| Perfume 6 | | | | | | 0.5 | | | | | |
| Perfume 7 | | | | | | | 0.5 | | | | |
| Perfume 8 | | | | | | | | 0.5 | | | |
| Perfume 9 | | | | | | | | | 0.5 | | |
| Perfume 10 | | | | | | | | | | 0.5 | |
| Perfume 11 | | | | | | | | | | | 0.5 |
| Perfume 12 | | | | | | | | | | | |
| Perfume 13 | | | | | | | | | | | |
| Perfume 14 | | | | | | | | | | | |
| Perfume 15 | | | | | | | | | | | |
| Perfume 16 | | | | | | | | | | | |
| Perfume 17 | | | | | | | | | | | |
| Perfume 18 | | | | | | | | | | | |
| Perfume 19 | | | | | | | | | | | |
| Perfume 20 | | | | | | | | | | | |
| Perfume 21 | | | | | | | | | | | |
| Perfume 22 | | | | | | | | | | | |
| Evaluation of fragrance | 4.5 | 3.5 | 3.0 | 4.5 | 4.0 | 4.5 | 4.5 | 4.5 | 4.5 | 4.5 | 4.5 |

[Table 9]

| | Example 12 | Example 13 | Example 14 | Example 15 | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|---|---|
| Composition in Table (Basic formulation) | 99.5 | 99.5 | 99.5 | 99.5 | 99.5 | 99.5 | 99.5 | 99.5 | 99.5 | 99.5 | 99.5 |
| Perfume 1 | | | | | | | | | | | |
| Perfume 2 | | | | | | | | | | | |
| Perfume 3 | | | | | | | | | | | |
| Perfume 4 | | | | | | | | | | | |
| Perfume 5 | | | | | | | | | | | |
| Perfume 6 | | | | | | | | | | | |
| Perfume 7 | | | | | | | | | | | |
| Perfume 8 | | | | | | | | | | | |
| Perfume 9 | | | | | | | | | | | |
| Perfume 10 | | | | | | | | | | | |
| Perfume 11 | | | | | | | | | | | |
| Perfume 12 | 0.5 | | | | | | | | | | |
| Perfume 13 | | 0.5 | | | | | | | | | |
| Perfume 14 | | | 0.5 | | | | | | | | |
| Perfume 15 | | | | 0.5 | | | | | | | |
| Perfume 16 | | | | | 0.5 | | | | | | |
| Perfume 17 | | | | | | 0.5 | | | | | |
| Perfume 18 | | | | | | | 0.5 | | | | |
| Perfume 19 | | | | | | | | 0.5 | | | |
| Perfume 20 | | | | | | | | | 0.5 | | |
| Perfume 21 | | | | | | | | | | 0.5 | |
| Perfume 22 | | | | | | | | | | | 0.5 |
| Evaluation of fragrance | 3.5 | 4.0 | 4.5 | 4.5 | 1.5 | 1.5 | 1.5 | 2.0 | 2.0 | 2.0 | 1.0 |

[Formulation Example]

The self-tanning cosmetics of the following Formulation Examples 1 and 2 are scented with 0.5 mass% of Perfume 3 shown in Table 6.

[Table 10]

| Formulation Example 1 (Ingredient) | mass% |
|---|---|
| Glycerin | 7.000 |
| Carbopol® ETD2020 * | 0.100 |
| Methylparaben | 0.200 |
| Sodium Hydroxide | 0.060 |
| Cetearyl Alcohol | 3.300 |
| Steareth-2 ** | 1.500 |
| Petrolatum | 2.000 |
| Stearic Acid | 0.500 |
| Dibutylhydroxytoluene | 0.100 |
| Dimethicone | 1.000 |
| Ethylhexyl Methoxycinnamate | 6.000 |
| Citric Acid | 0.100 |
| Erythrulose | 0.500 |
| Dihydroxyacetone | 2.000 |
| Waxy Corn Starch *** | 3.500 |
| Caramel | 0.020 |
| Water | 72.120 |
| Total | 100.000 |
| * Carbopol® EDT2020:Acrylates / C10-30 alkyl acrylate ..crosspolymer<br>** Steareth-2 is the polyethylene glycol ether of Stearyl Alcohol, wherein the average chain length of polyethylene glycol unit is 2.<br>*** Proposed INCI Name. Purchased from Akzo Nobel as a Trade Name of PC-10-40. | |

[Table 11]

| Formulation Example 2 (Ingredient) | mass% |
|---|---|
| Methylparaben | 0.300 |
| Glycerin | 2.000 |
| Xanthan Gum | 0.100 |
| Gryceryl Stearate | 1.000 |
| Steareth-2 * | 0.250 |
| Cetyl Alcohol | 0.250 |
| Behenyl Alcohol | 0.500 |
| Dimethicone | 0.500 |
| Phenyl Trimethicone | 0.500 |
| Polyacrylamide | 1.500 |

(continued)

| Formulation Example 2 (Ingredient) | mass% |
|---|---|
| Ethylhexyl Methoxycinnamate | 6.000 |
| Citric Acid | 0.040 |
| Erythrulose | 0.500 |
| Dihydroxyacetone | 2.000 |
| Waxy Corn Starch ** | 3.500 |
| Caramel | 0.020 |
| Water | 81.040 |
| Total | 100.000 |
| * Steareth-2 is the polyethylene glycol ether of Stearyl Alcohol, wherein the average chain length of polyethylene glycol unit is 2.<br>** Proposed INCI Name. Purchased from Akzo Nobel as a Trade Name of PC-10-40. | |

## Claims

1. A self-tanning cosmetic comprising the following component (A), component (B) and component (C):

(A) dihydroxyacetone;
(B) at least one or more compounds selected from the group consisting of 4-methyl-3-decen-5-ol, 2-ethyl-4-(2,2,3-trimethyl-3-cyclopenten-1-yl)-2-buten-1-ol, 2-hexyl-3-phenyl-2-propenal and 2-methyl-3-(4-iso-propylphenyl)propanal; and
(C) at least one or more compounds selected from the group consisting of 3,7-dimethyl-1,6-nonadien-3-ol, 1-(2,2,6-trimethyl-2-cyclohexenyl)-2-buten-1-one, 1-(5,5-dimethyl-1-cyclohexen-1-yl)-4-penten-1-one, 1-(3,3-dimethyl-1-cyclohexen-1-yl)-4-penten-1-one, benzaldehyde and gamma-nonalactone.

2. The self-tanning cosmetic according to Claim 1, wherein the mass ratio of the content of the component (B) and the component (C) is from 10:1 to 1:10.

3. The self-tanning cosmetic according to Claim 1 or 2, wherein the content of the component (B) is from 0.001 to 0.1 mass% of the cosmetic.

4. The self-tanning cosmetic according to any one of Claims 1 to 3, wherein the content of the component (C) is from 0.001 to 0.1 mass% of the cosmetic.

5. The self-tanning cosmetic according to any one of Claims 1 to 4, wherein the content of the component (A) is from 0.01 to 10 mass% of the cosmetic.

6. The self-tanning cosmetic according to any one of Claims 1 to 5, further comprising a polysaccharide as a component (E).

7. The self-tanning cosmetic according to Claim 6, wherein the content of the component (E) is from 0.1 to 10 mass%, preferably from 1 to 10 mass%, more preferably from 2 to 8 mass%, even more preferably from 3 to 7 mass%.

8. The self-tanning cosmetic according to any one of Claims 1 to 7, further comprising erythrulose as a component (F).

9. The self-tanning cosmetic according to Claim 8, wherein the total content of the components (A) and (F) is preferably from 0.01 to 20 mass%, more preferably from 0.01 to 15 mass%, even more preferably from 0.01 to 10 mass%, even more preferably from 0.01 to 7 mass%, even more preferably from 0.01 to 5 mass%.

10. A method for reducing odor derived from a compound represented by a formula (1) during application of a self-tanning cosmetic, comprising a step of application of a self-tanning cosmetic to the skin for a browning reaction, wherein the self-tanning cosmetic comprises:

(A) dihydroxyacetone;
(B) at least one or more compounds selected from the group consisting of 4-methyl-3-decen-5-ol, 2-ethyl-4-(2,2,3-trimethyl-3-cyclopenten-1-yl)-2-buten-1-ol, 2-hexyl-3-phenyl-2-propenal and 2-methyl-3-(4-iso-propylphenyl)propanal; and
(C) at least one or more compounds selected from the group consisting of 3,7-dimethyl-1,6-nonadien-3-ol, 1-(2,2,6-trimethyl-2-cyclohexenyl)-2-buten-1-one, 1-(5,5-dimethyl-1-cyclohexen-1-yl)-4-penten-1-one, 1-(3,3-dimethyl-1-cyclohexen-1-yl)-4-penten-1-one, benzaldehyde and gamma-nonalactone;

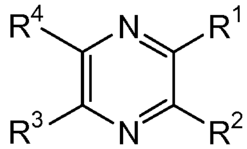

(1)

wherein $R^1$ represents a methyl group, an ethyl group or an acetyl group, and $R^2$, $R^3$ and $R^4$ each independently represent a hydrogen atom or a methyl group.

11. The method according to Claim 10, wherein the odor derived from the compound represented by the formula (1) is

an earthy odor.

12. Use of a composition comprising the following component (A), component (B) and component (C) as a self-tanning cosmetic:

(A) dihydroxyacetone;
(B) at least one or more compounds selected from the group consisting of 4-methyl-3-decen-5-ol, 2-ethyl-4-(2,2,3-trimethyl-3-cyclopenten-1-yl)-2-buten-1-ol, 2-hexyl-3-phenyl-2-propenal and 2-methyl-3-(4-iso-propylphenyl)propanal; and
(C) at least one or more compounds selected from the group consisting of 3,7-dimethyl-1,6-nonadien-3-ol, 1-(2,2,6-trimethyl-2-cyclohexenyl)-2-buten-1-one, 1-(5,5-dimethyl-1-cyclohexen-1-yl)-4-penten-1-one, 1-(3,3-dimethyl-1-cyclohexen-1-yl)-4-penten-1-one, benzaldehyde and gamma-nonalactone.

13. The use according to Claim 12, wherein the mass ratio of the contents of the component (B) and the component (C) in the composition is from 10:1 to 1:10.

14. The use according to Claim 12 or 13, wherein the content of the component (B) in the composition is from 0.001 to 0.1.

15. The use according to any one of Claims 12 to 14, wherein the content of the component (C) in the composition is from 0.001 to 0.1 mass%.

16. The use according to any one of Claims 12 to 15, for reducing odor derived from a compound according to formula (1) in a self-tanning method.

## Patentansprüche

1. Selbstbräunendes Kosmetikum, umfassend die nachstehende Komponente (A), Komponente (B) und Komponente (C):

(A) Dihydroxyaceton;
(B) zumindest eine oder mehrere Verbindungen, ausgewählt aus der Gruppe bestehend aus 4-Methyl-3-decen-5-ol, 2-Ethyl-4-(2,2,3-trimethyl-3-cyclopenten-l-yl)-2-buten-l-ol, 2-Hexyl-3-phenyl-2-propenal und 2-Methyl-3-(4-iso-propylphenyl)propanal; und
(C) zumindest eine oder mehrere Verbindungen, ausgewählt aus der Gruppe bestehend aus 3,7-Dimethyl-1,6-nonadien-3-ol, 1-(2,2,6-Trimethyl-2-cyclohexenyl)-2-buten-1-on, 1-(5,5-Dimethyl-1-cyclohexen-1-yl)-4-penten-1-on, 1-(3,3-Dimethyl-1-cyclohexen-1-yl)-4-penten-1-on, Benzaldehyd und gamma-Nonalacton.

2. Selbstbräunendes Kosmetikum gemäss Anspruch 1, wobei das Masseverhältnis des Gehalts der Komponente (B) und der Komponente (C) 10:1 bis 1:10 beträgt.

3. Selbstbräunendes Kosmetikum gemäss Anspruch 1 oder 2, wobei der Gehalt der Komponente (B) 0,001 bis 0,1 Masse-% des Kosmetikums beträgt.

4. Selbstbräunendes Kosmetikum gemäss irgendeinem der Ansprüche 1 bis 3, wobei der Gehalt der Komponente (C) 0,001 bis 0,1 Masse-% des Kosmetikums beträgt.

5. Selbstbräunendes Kosmetikum gemäss irgendeinem der Ansprüche 1 bis 4, wobei der Gehalt der Komponente (A) 0,01 bis 10 Masse-% des Kosmetikums beträgt.

6. Selbstbräunendes Kosmetikum gemäss irgendeinem der Ansprüche 1 bis 5, die ferner ein Polysaccharid als Komponente (E) umfasst.

7. Selbstbräunendes Kosmetikum gemäss Anspruch 6, wobei der Gehalt der Komponente (E) 0,1 bis 10 Masse-%, vorzugsweise 1 bis 10 Masse-%, stärker bevorzugt 2 bis 8 Masse-%, noch stärker bevorzugt 3 bis 7 Masse-% beträgt.

8. Selbstbräunendes Kosmetikum gemäss irgendeinem der Ansprüche 1 bis 7, die ferner Erythrulose als Komponente (F) umfasst.

**9.** Selbstbräunendes Kosmetikum gemäss Anspruch 8, wobei der Gesamtgehalt der Komponenten (A) und (F) vorzugsweise 0,01 bis 20 Masse-%, stärker bevorzugt 0,01 bis 15 Masse-%, noch stärker bevorzugt 0,01 bis 10 Masse-%, noch stärker bevorzugt 0,01 bis 7 Masse-%, noch stärker bevorzugt 0,01 bis 5 Masse-% beträgt.

**10.** Verfahren zur Reduzierung des von einer Verbindung der Formel (1) stammenden Geruchs während des Auftragens eines selbstbräunenden Kosmetikums, umfassend einen Schritt zum Auftragen eines selbstbräunenden Kosmetikums auf die Haut für eine Bräunungsreaktion, wobei das selbstbräunende Kosmetikum umfasst:

(A) Dihydroxyaceton;
(B) zumindest eine oder mehrere Verbindungen, ausgewählt aus der Gruppe bestehend aus 4-Methyl-3-decen-5-ol, 2-Ethyl-4-(2,2,3-trimethyl-3-cyclopenten-l-yl)-2-buten-l-ol, 2-Hexyl-3-phenyl-2-propenal und 2-Methyl-3-(4-iso-propylphenyl)propanal; und
(C) zumindest eine oder mehrere Verbindungen, ausgewählt aus der Gruppe bestehend aus 3,7-Dimethyl-1,6-nonadien-3-ol, 1-(2,2,6-Trimethyl-2-cyclohexenyl)-2-buten-1-on, 1-(5,5-Dimethyl-1-cyclohexen-1-yl)-4-penten-1-on, 1-(3,3-Dimethyl-1-cyclohexen-1-yl)-4-penten-1-on, Benzaldehyd und gamma-Nonalacton;

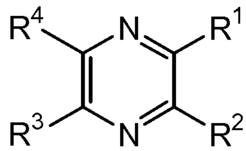

(1)

worin $R^1$ eine Methylgruppe, eine Ethylgruppe oder eine Acetylgruppe darstellt und $R^2$, $R^3$ und $R^4$ jeweils unabhängig ein Wasserstoffatom oder eine Methylgruppe darstellen.

**11.** Verfahren gemäss Anspruch 10, wobei der von der Verbindung der Formel (1) stammende Geruch ein erdiger Geruch ist.

**12.** Verwendung einer Zusammensetzung, umfassend die nachstehende Komponente (A), Komponente (B) und Komponente (C), als selbstbräunendes Kosmetikum:

(A) Dihydroxyaceton;
(B) zumindest eine oder mehrere Verbindungen, ausgewählt aus der Gruppe bestehend aus 4-Methyl-3-decen-5-ol, 2-Ethyl-4-(2,2,3-trimethyl-3-cyclopenten-l-yl)-2-buten-l-ol, 2-Hexyl-3-phenyl-2-propenal und 2-Methyl-3-(4-iso-propylphenyl)propanal; und
(C) zumindest eine oder mehrere Verbindungen, ausgewählt aus der Gruppe bestehend aus 3,7-Dimethyl-1,6-nonadien-3-ol, 1-(2,2,6-Trimethyl-2-cyclohexenyl)-2-buten-1-on, 1-(5,5-Dimethyl-1-cyclohexen-1-yl)-4-penten-1-on, 1-(3,3-Dimethyl-1-cyclohexen-1-yl)-4-penten-1-on, Benzaldehyd und gamma-Nonalacton.

**13.** Verwendung gemäss Anspruch 12, wobei das Masseverhältnis des Gehalts der Komponente (B) und der Komponente (C) in der Zusammensetzung 10:1 bis 1:10 beträgt.

**14.** Verwendung gemäss Anspruch 12 oder 13, wobei der Gehalt der Komponente (B) in der Zusammensetzung 0,001 bis 0,1 beträgt.

**15.** Verwendung gemäss irgendeinem der Ansprüche 12 bis 14, wobei der Gehalt der Komponente (C) in der Zusammensetzung 0,001 bis 0,1 Masse-% beträgt.

**16.** Verwendung gemäss irgendeinem der Ansprüche 12 bis 15, zum Reduzieren des Geruchs, der von einer Verbindung der Formel (1) stammt, in einem Selbstbräunungsverfahren.

## Revendications

**1.** Cosmétique auto-bronzant comprenant le composant (A), le composant (B) et le composant (C) suivants :

(A) la dihydroxyacétone ;
(B) au moins un ou plusieurs composé(s) choisi(s) dans le groupe constitué par le 4-méthyl-3-décèn-5-ol, le 2-éthyl-4-(2,2,3-triméthyl-3-cyclopentèn-1-yl)-2-butèn-1-ol, le 2-hexyl-3-phényl-2-propénal et le 2-méthyl-3-(4-iso-propylphényl)propanal ; et
(C) au moins un ou plusieurs composé(s) choisi(s) dans le groupe constitué par le 3,7-diméthyl-1, 6-nonadièn-3-ol, le 1-(2,2,6-triméthyl-2-cyclohexényl)-2-butèn-1-one, le 1-(5,5-diméthyl-1-cyclohexèn-1-yl)-4-pentèn-1-one, le 1- (3, 3-diméthyl-1-cyclohexèn-1-yl)-4-pentèn-1-one, le benzaldéhyde et une gamma-non-lactone.

**2.** Cosmétique auto-bronzant selon la revendication 1, dans lequel le rapport massique de la teneur en composant (B) et en composant (C) est de 10:1 à 1:10.

**3.** Cosmétique auto-bronzant selon la revendication 1 ou 2, dans lequel la teneur en composant (B) est de 0,001 à 0,1 % en masse du cosmétique.

**4.** Cosmétique auto-bronzant selon l'une quelconque des revendications 1 à 3, dans lequel la teneur en composant (C) est de 0,001 à 0,1 % en masse du cosmétique.

**5.** Cosmétique auto-bronzant selon l'une quelconque des revendications 1 à 4, dans lequel la teneur en composant (A) est de 0,01 à 10 % en masse du cosmétique.

**6.** Cosmétique auto-bronzant selon l'une quelconque des revendications 1 à 5, comprenant en outre un polysaccharide en tant que composant (E).

**7.** Cosmétique auto-bronzant selon la revendication 6, dans lequel la teneur en composant (E) est de 0,1 à 10 % en masse, de préférence de 1 à 10 % en masse, plus de préférence de 2 à 8 % en masse, encore plus de préférence de 3 à 7 % en masse.

**8.** Cosmétique auto-bronzant selon l'une quelconque des revendications 1 à 7, comprenant en outre de l'érythrulose en tant que composant (F).

**9.** Cosmétique auto-bronzant selon la revendication 8, dans lequel la teneur totale en composants (A) et (F) est de préférence de 0,01 à 20 % en masse, plus de préférence de 0,01 à 15 % en masse, encore plus de préférence de 0,01 à 10% en masse, encore plus de préférence de 0,01 à 7 % en masse, encore plus de préférence de 0,01 à 5 % en masse.

**10.** Procédé pour réduire une odeur dérivée d'un composé représenté par une formule (1) durant l'application d'un cosmétique auto-bronzant, comprenant une étape d'application d'un cosmétique autobronzant sur la peau pour une réaction de brunissement, dans lequel le cosmétique autobronzant comprend :

(A) la dihydroxyacétone ;
(B) au moins un ou plusieurs composé(s) choisi(s) dans le groupe constitué par le 4-méthyl-3-décèn-5-ol, le 2-éthyl-4-(2,2,3-triméthyl-3-cyclopentèn-1-yl)-2-butèn-1-ol, le 2-hexyl-3-phényl-2-propénal et le 2-méthyl-3-(4-iso-propylphényl)propanal ; et
(C) au moins un ou plusieurs composé(s) choisi(s) dans le groupe constitué par le 3,7-diméthyl-1, 6-nonadièn-3-ol, le 1-(2,2,6-triméthyl-2-cyclohexényl)-2-butèn-1-one, le 1-(5,5-diméthyl-1-cyclohexèn-1-yl)-4-pentèn-1-one, le 1-(3, 3-diméthyl-1-cyclohexèn-1-yl)-4-pentèn-1-one, le benzaldéhyde et une gamma-non-lactone ;

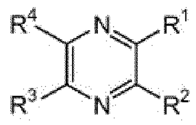

(1)

dans laquelle $R^1$ représente un groupe méthyle, un groupe éthyle ou un groupe acétyle et $R^2$, $R^3$ et $R^4$ représentent chacun indépendamment un atome d'hydrogène ou un groupe

méthyle.

**11.** Procédé selon la revendication 10, dans lequel l'odeur dérivée du composé représenté par la formule (1) est une odeur de terre.

**12.** Utilisation d'une composition comprenant le composant (A), le composant (B) et le composant (C) suivants en tant que cosmétique auto-bronzant :

(A) dihydroxyacétone ;
(B) au moins un ou plusieurs composé(s) choisi(s) dans le groupe constitué par le 4-méthyl-3-décèn-5-ol, le 2-éthyl-4-(2,2,3-triméthyl-3-cyclopentèn-1-yl)-2-butèn-1-ol, le 2-hexyl-3-phényl-2-propénal et le 2-méthyl-3-(4-iso-propylphényl)propanal ; et
(C) au moins un ou plusieurs composé(s) choisi(s) dans le groupe constitué par le 3,7-diméthyl-1, 6-nonadièn-3-ol, le 1-(2,2,6-triméthyl-2-cyclohexényl)-2-butèn-1-one, le 1-(5,5-diméthyl-1-cyclohexèn-1-yl)-4-pentèn-1-one, le 1-(3, 3-diméthyl-1-cyclohexèn-1-yl)-4-pentèn-1-one, le benzaldéhyde et une gamma-non-lactone.

**13.** Utilisation selon la revendication 12, dans laquelle le rapport massique des teneurs du composant (B) et du composant (C) dans la composition est de 10 :1 à 1 :10.

**14.** Utilisation selon la revendication 12 ou 13, dans laquelle la teneur du composant (B) dans la composition est de 0,001 à 0,1.

**15.** Utilisation selon l'une quelconque des revendications 12 à 14, dans laquelle la teneur du composant (C) dans la composition est de 0,001 à 0,1 % en masse.

**16.** Utilisation selon l'une quelconque des revendications 12 à 15, pour réduire une odeur dérivée d'un composé selon la formule (1) dans un procédé d'auto-bronzage.

Fig. 1

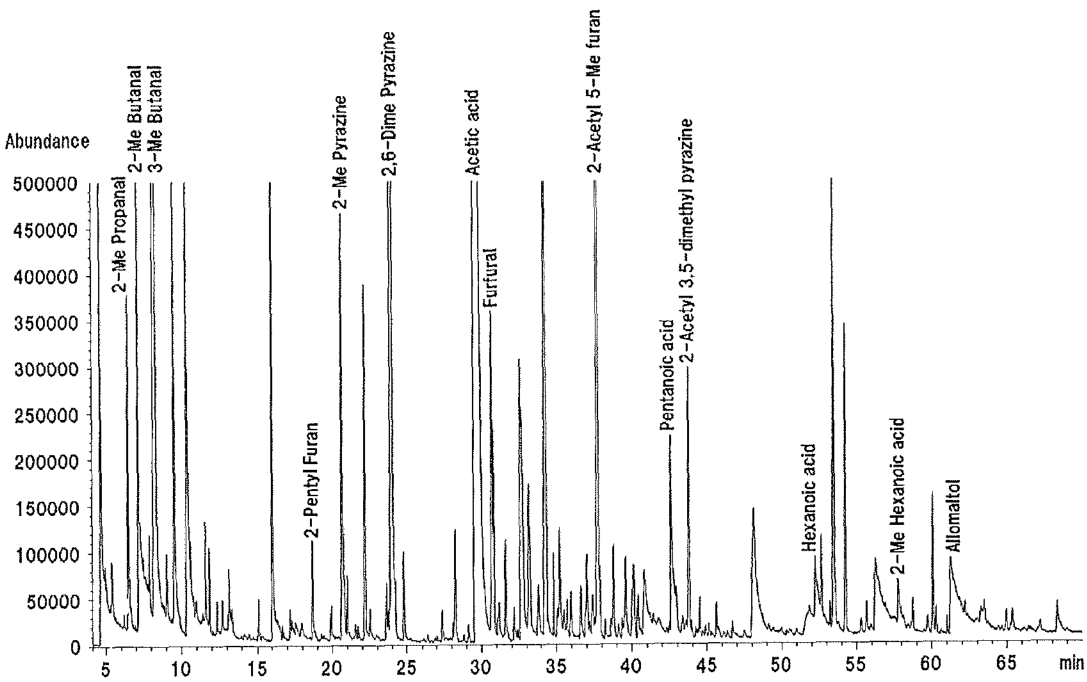
Abundance
500000
450000
400000
350000
300000
250000
200000
150000
100000
50000
0
5
10
15
20
25
30
35
40
45
50
55
60
65
min
2-Me Propanal
2-Me Butanal
3-Me Butanal
2-Pentyl Furan
2-Me Pyrazine
2,6-Dime Pyrazine
Acetic acid
Furfural
2-Acetyl 5-Me furan
Pentanoic acid
2-Acetyl 3,5-dimethyl pyrazine
Hexanoic acid
2-Me Hexanoic acid
Allomaltol

Fig. 2

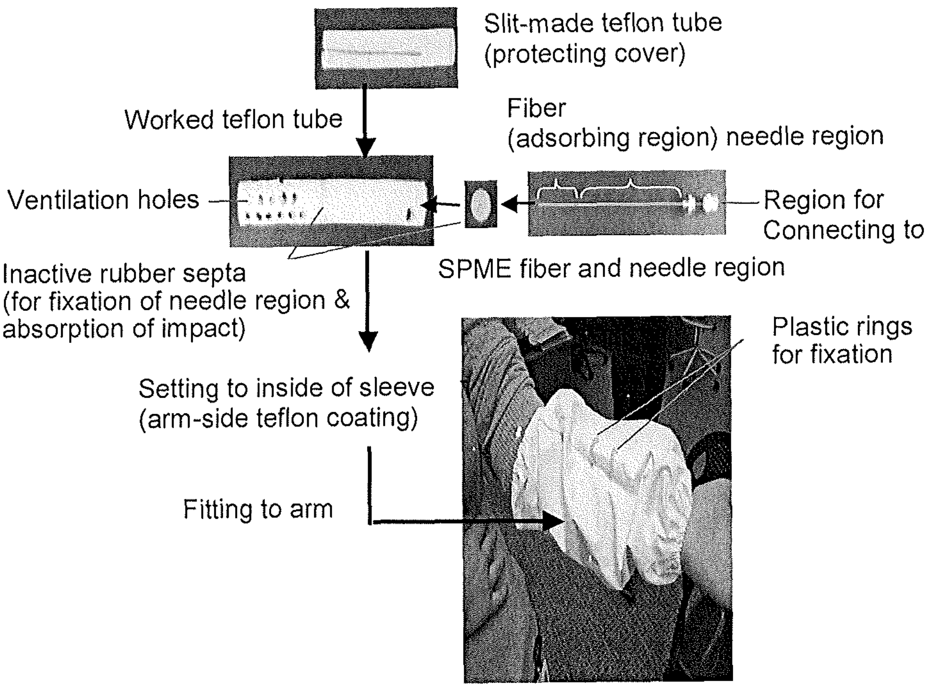
Slit-made teflon tube
(protecting cover)
Worked teflon tube
Fiber
(adsorbing region) needle region
Ventilation holes
Region for
Connecting to
Inactive rubber septa
(for fixation of needle region &
absorption of impact)
SPME fiber and needle region
Plastic rings
for fixation
Setting to inside of sleeve
(arm-side teflon coating)
Fitting to arm

Fig. 3

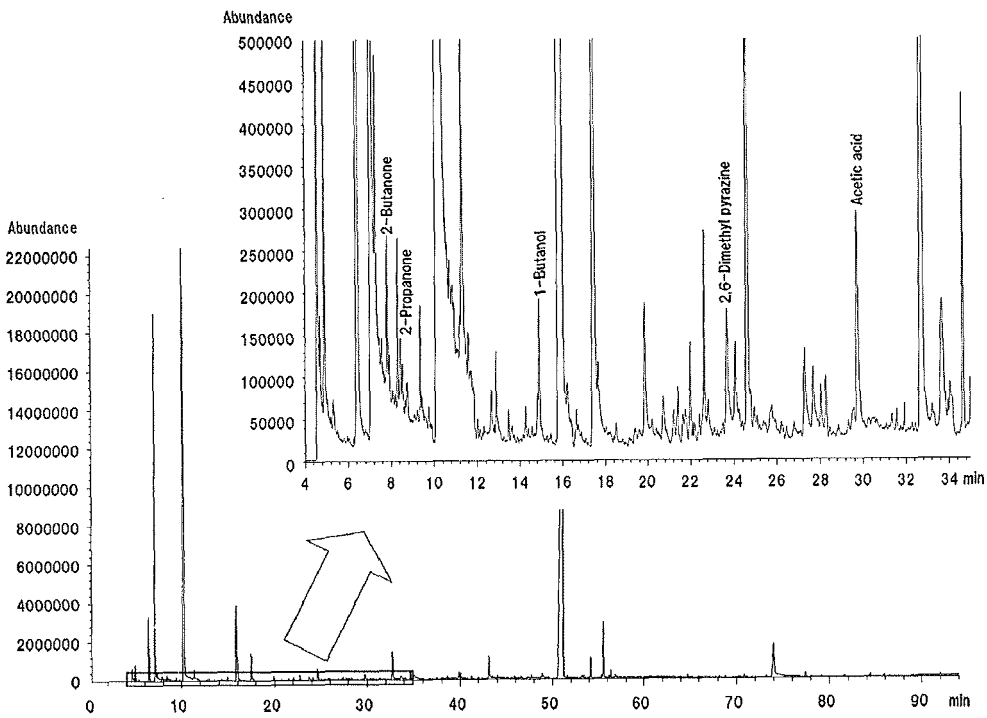
Abundance
500000
450000
400000
350000
300000
250000
200000
150000
100000
50000
0
4 6 8 10 12 14 16 18 20 22 24 26 28 30 32 34 min
2-Butanone
2-Propanone
1-Butanol
2,6-Dimethyl pyrazine
Acetic acid
Abundance
22000000
20000000
18000000
16000000
14000000
12000000
10000000
8000000
6000000
4000000
2000000
0
0 10 20 30 40 50 60 70 80 90 min

Fig. 4

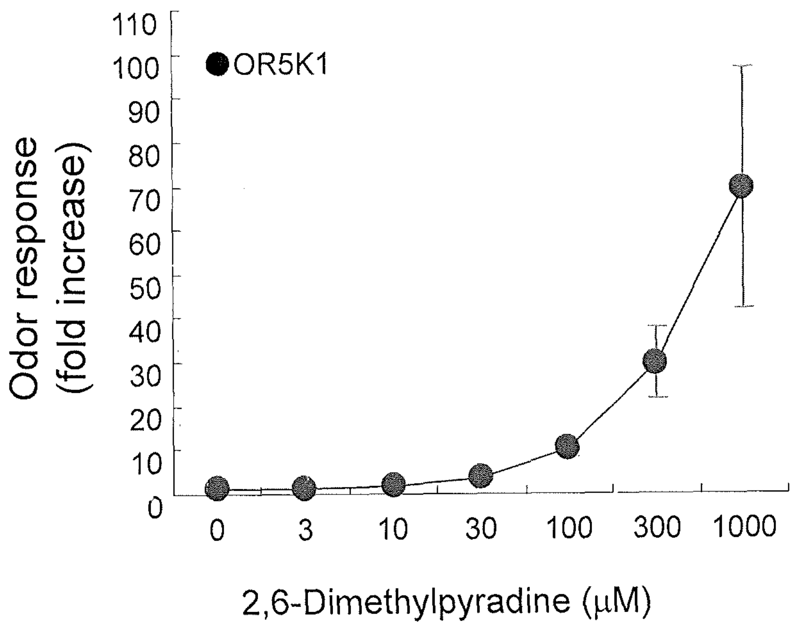
OR5K1
Odor response
(fold increase)
110
100
90
80
70
60
50
40
30
20
10
0
0 3 10 30 100 300 1000
2,6-Dimethylpyradine (μM)

Fig. 5

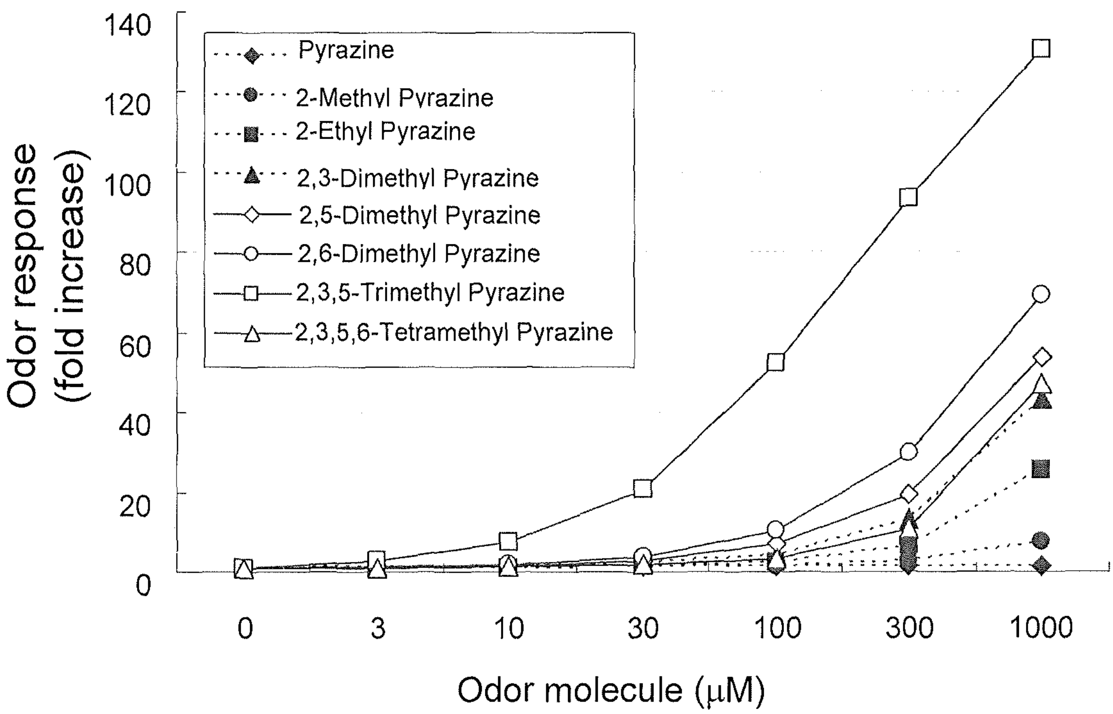
Pyrazine
2-Methyl Pyrazine
2-Ethyl Pyrazine
2,3-Dimethyl Pyrazine
2,5-Dimethyl Pyrazine
2,6-Dimethyl Pyrazine
2,3,5-Trimethyl Pyrazine
2,3,5,6-Tetramethyl Pyrazine
Odor response (fold increase)
140
120
100
80
60
40
20
0
0
3
10
30
100
300
1000
Odor molecule (μM)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 02096371 A
- US 6086903 A
- US 20110152383 A
- EP 1719554 A
- US 61636093 B

### Non-patent literature cited in the description

- **D. M. HINDENLANG ; M. E. MCDONNELL.** *Cosmetics & Toiletries magazine,* 2008, vol. 123 (7), 67-74
- Common Fragrance and Flavor Materials. **HORST SURBURG ; JOHANNES PANTEN.** Preparation, Properties and Uses. John Wiley & Sons, 2006
- **STEFFEN ARCTANDER.** Perfume and Flavor Chemicals. 1969, vol. 1, 2
- **STEFFEN ARCTANDER.** Perfume and Flavor Materials of Natural Origin. 1961

SEQUENCE LISTING

<110> Kao Corporation

<120> Selftanning cosmetics

<130> KS1194

<150> US 61/636,093
<151> 2012-04-20

<160> 4

<170> PatentIn version 3.1

<210> 1
<211> 927
<212> DNA
<213> Homo sapiens OR5K1

<400> 1

atggctgaag aaaatcatac catgaaaaat gagtttatcc tcacaggatt

tacagatcac 60

cctgagctga agactctgct gtttgtggtg ttctttgcca tctatctgat

caccgtggtg 120

gggaatatta gtttggtggc actgatattt acacaccgtc ggcttcacac accaatgtac 180

atctttctgg gaaatctggc tcttgtggat tcttgctgtg cctgtgctat tacccccaaa 240

atgttagaga acttcttttc tgagaacaaa aggatttccc tctatgaatg tgcagtacag 300

ttttattttc tttgcactgt ggaaactgca gactgctttc ttctggcagc aatggcctat 360

gaccgctatg tggccatatg caacccactg cagtaccaca tcatgatgtc caagaaactc 420

tgcattcaga tgaccacagg ggccttcata gctggaaacc tgcattccat gattcatgta 480

gggcttgtat ttaggttagt tttctgtgga tcgaatcaca tcaaccactt ttactgtgat 540

attcttccct tgtatagact ctcttgtgtt gatccttata tcaatgaact ggttctattc 600

atcttctcag gttcagttca agtctttacc ataggtagtg tcttaatatc ttatctctat 660

attcttctta ctattttcaa aatgaaatcc aaagagggaa gggccaaagc tttttctacc 720

tgtgcatccc actttttgtc agtttcatta ttctatggat ctcttttctt catgtacgtt 780

agaccaaatt tgcttgaaga aggggataaa gatataccag ctgcaatttt atttacaata 840

gtagttccct tactaaatcc tttcatttat agcctgagaa atagggaagt aataagtgtc 900

ttaagaaaaa ttctgatgaa gaaataa 927

<210> 2
<211> 308
<212> PRT
<213> Homo sapiens OR5K1

<400> 2

Met Ala Glu Glu Asn His Thr Met Lys Asn Glu Phe Ile Leu Thr Gly
1 5 10 15

Phe Thr Asp His Pro Glu Leu Lys Thr Leu Leu Phe Val Val Phe Phe

20 25 30

Ala Ile Tyr Leu Ile Thr Val Val Gly Asn Ile Ser Leu Val Ala Leu

35 40 45

Ile Phe Thr His Arg Arg Leu His Thr Pro Met Tyr Ile Phe Leu Gly

50 55 60

Asn Leu Ala Leu Val Asp Ser Cys Cys Ala Cys Ala Ile Thr Pro Lys

65 70 75 80

Met Leu Glu Asn Phe Phe Ser Glu Asn Lys Arg Ile Ser Leu Tyr Glu

85 90 95

Cys Ala Val Gln Phe Tyr Phe Leu Cys Thr Val Glu Thr Ala Asp Cys

100 105 110

Phe Leu Leu Ala Ala Met Ala Tyr Asp Arg Tyr Val Ala Ile Cys Asn

115 120 125

Pro Leu Gln Tyr His Ile Met Met Ser Lys Lys Leu Cys Ile Gln Met

130 135 140

Thr Thr Gly Ala Phe Ile Ala Gly Asn Leu His Ser Met Ile His Val

145 150 155 160

Gly Leu Val Phe Arg Leu Val Phe Cys Gly Ser Asn His Ile Asn His

165 170 175

Phe Tyr Cys Asp Ile Leu Pro Leu Tyr Arg Leu Ser Cys Val Asp Pro

180 185 190

Tyr Ile Asn Glu Leu Val Leu Phe Ile Phe Ser Gly Ser Val Gln Val

195 200 205

Phe Thr Ile Gly Ser Val Leu Ile Ser Tyr Leu Tyr Ile Leu Leu Thr

210 215 220

Ile Phe Lys Met Lys Ser Lys Glu Gly Arg Ala Lys Ala Phe Ser Thr

225 230 235 240

Cys Ala Ser His Phe Leu Ser Val Ser Leu Phe Tyr Gly Ser Leu Phe

245 250 255

Phe Met Tyr Val Arg Pro Asn Leu Leu Glu Glu Gly Asp Lys Asp Ile

260 265 270

Pro Ala Ala Ile Leu Phe Thr Ile Val Val Pro Leu Leu Asn Pro Phe

275 280 285

Ile Tyr Ser Leu Arg Asn Arg Glu Val Ile Ser Val Leu Arg Lys Ile

290 295 300

Leu Met Lys Lys

305

<210> 3
<211> 681
<212> DNA
<213> Homo sapiens RTP1S

<400> 3

atgtgcaagt ccctgacaac gggagagtgg aagaagatct tctacgagaa aatggaggag 60

gtgaaacccg cagactcctg ggacctgatc atggatccca acctccagca taacgtattg 120

gcccccggat ggaagcagta cctggagcag cacgcctctg gccgcttcca ctgctcctgg 180

tgctggcata gctggcagtc ctcccaactg gtgatcctct tccacatgta cctggataag 240

acccagcgga cgggctgcgt gcgcatgaga gtcttcaagc agctctgcta cgagtgtggc 300

tcctcccggc tggacgagtc gtccatgctg gaggagaaca tagaggggct ggtggacaac 360

ctcgtctgca gcctccggga gcagtgctac ggggagaatg gggacagta ccgcatccac 420

gtggcctccc gccaagacca ccagcgccac cggggagagt tctgcgaggc ctgccgcctg 480

ggcatcaccc actggaagcc cacggagaag atgctagagg aggaggcctc cacctacacc 540

ttctcccggc ctgcgaatcc ttccaagaca gccgactcgg gtttcagctg tgacttctgc 600

tccctccctt ggtgtatgtt ctgggccacg gtgctcttgc tcatcatata cctgcagatc 660

tccttcggca accctgtcta a 681

<210> 4
<211> 226
<212> PRT
<213> Homo sapiens RTP1S

<400> 4

Met Cys Lys Ser Leu Thr Thr Gly Glu Trp Lys Lys Ile Phe Tyr Glu
1 5 10 15

Lys Met Glu Glu Val Lys Pro Ala Asp Ser Trp Asp Leu Ile Met Asp
20 25 30

Pro Asn Leu Gln His Asn Val Leu Ala Pro Gly Trp Lys Gln Tyr Leu
35 40 45

Glu Gln His Ala Ser Gly Arg Phe His Cys Ser Trp Cys Trp His Ser

50 55 60

Trp Gln Ser Ser Gln Leu Val Ile Leu Phe His Met Tyr Leu Asp Lys

65 70 75 80

Thr Gln Arg Thr Gly Cys Val Arg Met Arg Val Phe Lys Gln Leu Cys

85 90 95

Tyr Glu Cys Gly Ser Ser Arg Leu Asp Glu Ser Ser Met Leu Glu Glu

100 105 110

Asn Ile Glu Gly Leu Val Asp Asn Leu Val Cys Ser Leu Arg Glu Gln

115 120 125

Cys Tyr Gly Glu Asn Gly Gly Gln Tyr Arg Ile His Val Ala Ser Arg

130 135 140

Gln Asp His Gln Arg His Arg Gly Glu Phe Cys Glu Ala Cys Arg Leu

145 150 155 160

Gly Ile Thr His Trp Lys Pro Thr Glu Lys Met Leu Glu Glu Glu Ala

165 170 175

Ser Thr Tyr Thr Phe Ser Arg Pro Ala Asn Pro Ser Lys Thr Ala Asp

180 185 190

Ser Gly Phe Ser Cys Asp Phe Cys Ser Leu Pro Trp Cys Met Phe Trp

195 200 205

Ala Thr Val Leu Leu Leu Ile Ile Tyr Leu Gln Ile Ser Phe Gly Asn

210 215 220

Pro Val

225